# EUROPEAN PATENT APPLICATION

(11) **EP 4 644 355 A1**
(43) Date of publication of application: **05.11.2025**
(21) Application number: 24305674.4
(22) Date of filing: 30.04.2024
(51) Int. Cl.: C07C 4/22, C07C 15/16, C07C 5/333, C07C 7/00, C07C 7/04

(54) **STYRENE MONOMER FROM FEEDSTOCK INCLUDING PYROLYSIS OIL**

(71) Applicant: T.EN Process Technology, Inc., Houston, TX 77079 (US)
(72) Inventor: Bishop, Jonathan, Houston, 77079 (US); Vallieres, Peter, Houston, 77079 (US)
(74) Representative: Edson, Russell Gregory

(57) **Abstract**

A system can be used to generate styrene monomer product from feedstock originating from a pyrolysis operation and a dehydrogenation operation. The system can include a styrene processing subsystem and a distillation column. The styrene processing subsystem can be positioned to receive first feedstock and to generate the styrene monomer product. The styrene processing subsystem can include an initial distillation column. The distillation column can be coupled with the initial distillation column to provide at least a portion of the first feedstock. The distillation column can positioned to receive second feedstock that can include pyrolysis oil and to generate the portion of the first feedstock by separating styrene from the pyrolysis oil.

## Description

### Technical Field

The present disclosure relates generally to chemical processing and, more particularly (although not necessarily exclusively), to purifying styrene from feedstock that can be generated from pyrolysis oil and a styrene dehydrogenation operation.

### Background

Environmental pollution, resource scarcity, climate change, and the like are contemporary problems that have been driving development of cyclical, or circular, economies to replace various linear economies. For example, processes can be used for recovering raw materials from plastic waste. Plastic waste is increasing and has various negative effects on the environment, on public health, on the economy, and the like. Not all thermoplastic polymers can be recycled at similar rates or efficiencies. Tailoring a chemical recycling process to specific polymers or classes of polymers can be difficult.

### Summary

In some examples, a system can be used to generate styrene monomer from feedstock that includes pyrolysis oil. The system can include a styrene processing subsystem and a second distillation column. The styrene processing subsystem can be positioned to receive first feedstock and to generate styrene monomer, and the styrene processing subsystem can include an initial distillation column. The second distillation column can be coupled with the initial distillation column to provide the initial distillation column with at least a portion of the first feedstock. The second distillation column can be positioned to receive second feedstock that can include a pyrolysis oil and to generate the portion of the first feedstock by separating styrene from the pyrolysis oil.

In some examples, a method can be used to generate styrene monomer from feedstock that includes pyrolysis oil. The method can include receiving, at a second distillation column of a styrene processing system, a first portion of a first feedstock. The first portion can include a pyrolysis oil. The method can include generating, by the second distillation column, second feedstock by distilling styrene from the first feedstock. The method can include receiving, by an initial distillation column of a styrene processing subsystem of the styrene processing system, a second portion of the first feedstock and the second feedstock. The method can include generating, by a distillation train that can include the initial distillation column, styrene monomer.

### Brief Description of the Drawings

FIG. 1 is a diagram of a styrene processing system that includes a distillation column for separating styrene from contaminants included in pyrolysis oil according to some examples of the present disclosure.
FIG. 2 is a diagram of a styrene processing system that includes a polishing column and a purification step for generating styrene monomer according to some examples of the present disclosure.
FIG. 3 is a flowchart of a process for generating styrene monomer using a styrene processing system that includes a distillation column for separating styrene from contaminants included in pyrolysis oil according to some examples of the present disclosure.
FIG. 4 is a flowchart of a process for generating styrene monomer using a styrene processing system that includes a polishing column and a purification step according to some examples of the present disclosure.

### Detailed Description

Certain aspects and features of the present disclosure relate to generating styrene monomer using a modified process flow that includes an additional distillation step that can allow feedstock generated from pyrolysis oil to be used. Styrene monomer may be or include a material having eight carbons and that can be used to make plastics, rubber, and the like. For example, the formula for styrene is *C*₈*H*₈. The pyrolysis oil may be generated from a pyrolysis operation that may convert waste plastics, such as waste polystyrene, into the pyrolysis oil that includes styrene and contaminants. The additional distillation step may be or include a stripper column or any other suitable distillation column that can receive the pyrolysis oil and distill, or otherwise separate, the contaminants from the styrene to produce a first portion of the feedstock, which can be provided to a styrene processing subsystem. The styrene processing subsystem may include one or more distillation steps that can be used to generate the styrene monomer. The feedstock provided to the styrene processing subsystem can include the first portion and a second portion that includes an output of an ethylbenzene dehydrogenation operation. The styrene processing subsystem can receive a combination of the first portion and the second portion as input and can generate the styrene monomer via one or more distillation processes, one or more purification processes, one or more polishing processes, other suitable processes, or any combination thereof.

Other styrene distillation units may be designed to receive feedstock including only material, such as dehydrogenation effluent, from ethylbenzene dehydrogenation operations. Adding pyrolysis oil to the other styrene distillation units can lead to high impurities in a final styrene product, which may render the final styrene product inadequate for use. Many compounds or contaminants present in the pyrolysis oil may also cause fouling in the other styrene distillation units.

Dehydrogenation effluent can include a high fraction of ethylbenzene, and separating the ethylbenzene from styrene is energy-intensive and requires columns with many theoretical stages. Separating styrene from ethylbenzene may be an energy-intensive process, but separating styrene from heavy impurities may be less energy-intensive compared with separating styrene from ethylbenzene. The pyrolysis process also may not separate benzene and toluene from ethylbenzene, which can be performed in a distillation subsystem, which may follow a dehydrogenation operation for recovering the ethylbenzene as a reactant.

A system can be used to generate styrene monomer from input feedstock that includes first feedstock originating from pyrolysis oil and second feedstock originating from a dehydrogenation operation. The system can include a distillation step that can be used to pre-process pyrolysis oil generated from a pyrolysis operation performed on waste plastics such as waste polystyrene. In some examples, the distillation step can be or include a stripper column that can strip the pyrolysis oil, for example to generate styrene and contaminants, prior to the pyrolysis oil being provided to a styrene processing subsystem, which may be or include a distillation train that can remove impurities from feedstock to generate the styrene monomer. In some examples, the styrene processing subsystem can additionally include a polishing step that removes further impurities, such as impurities introduced via the pyrolysis operation, from styrene product generated from the styrene processing subsystem.

The styrene processing subsystem can be augmented with a distillation step that can generate at least a portion of the feedstock into the styrene processing subsystem based on pyrolysis oil. The distillation step may involve a column that can achieve approximately ten theoretical stages, though other suitable numbers, such as less than ten or more than ten, of stages are possible for the distillation column. In some examples, the distillation column may operate with a bottom temperature from approximately 90 °C to approximately 155 °C, such as from approximately 100 °C to approximately 150 °C, and a top or head temperature from approximately 55 °C to approximately 100 °C, such as from approximately 65 °C to approximately 90 °C, and at a bottom pressure from approximately 40 mmHg to approximately 300 mmHg, such as from approximately 70 mmHg to approximately 200 mmHg, and a top or head pressure from approximately 40 mmHg to approximately 180 mmHg, such as from approximately 50 mmHg to approximately 120 mmHg. Pyrolysis oil from a polystyrene pyrolysis operation can be provided to the distillation column. A stream from an existing distillation plant, such as the styrene processing subsystem, can also be provided to the distillation column. Examples of sources for the stream can include crude styrene, recycled ethylbenzene, crude ethylbenzene and styrene after having toluene and lighter compounds distilled away, an unfinished styrene stream after having ethylbenzene and lighter compounds distilled away, styrene monomer, and the like. In some examples, the examples of the sources for the stream can be sources from the distillation subsystem, from the dehydrogenation system, or a combination thereof.

In some examples, the distillation step may be or include an initial distillation column in the system. The initial distillation column can be used to generate (i) tops, or overhead including first material and (ii) bottoms that can include second material. The second material, or the bottoms, can be reboiled, and product from the second material can be taken as residue. The first material, or the overhead, can be enriched in styrene with most or all high-boiling material removed. The first material can be provided, for example as vapor without condensation or as liquid after condensation, to a subsequent distillation column in the system. In some examples, the subsequent distillation column may be a first distillation column in a distillation train of the styrene processing subsystem.

In addition to the initial distillation step, the styrene processing subsystem can be augmented with a polishing step that may be or include another distillation column or other suitable type of polishing column. The polishing column may receive the styrene monomer product produced by the styrene processing subsystem and can distill away close-boiling compounds such as cumene. The polishing column can be packed or trayed such that the polishing column can achieve around 12 theoretical stages, though other suitable numbers, such as less than 12 or more than 12, of stages are possible for the polishing column. In some examples, the polishing column may operate with a bottom temperature from approximately 50 °C to approximately 100 °C, such as from approximately 60 °C to approximately 90 °C, and a top or head temperature from approximately 40 °C to approximately 90 °C, such as from approximately 50 °C to approximately 80 °C, and at a bottom pressure from approximately 40 mmHg to approximately 200 mmHg, such as from approximately 70 mmHg to approximately 150 mmHg, and a top or head pressure from approximately 30 mmHg to approximately 120 mmHg, such as from approximately 35 mmHg to approximately 100 mmHg. Bottoms generated by the polishing column can be reboiled and taken as residue. Overhead generated by the polishing column can be condensed, and the condensed overhead not used as reflux can be produced as styrene monomer product. The polishing column can allow close-boiling material, which may not have been sufficiently removed by the styrene processing subsystem, to be removed prior to generating the styrene monomer product.

These illustrative examples are given to introduce the reader to the general subject matter discussed here and are not intended to limit the scope of the disclosed concepts. The following sections describe various additional features and examples with reference to the drawings in which like numerals indicate like elements but, like the illustrative examples, should not be used to limit the present disclosure.

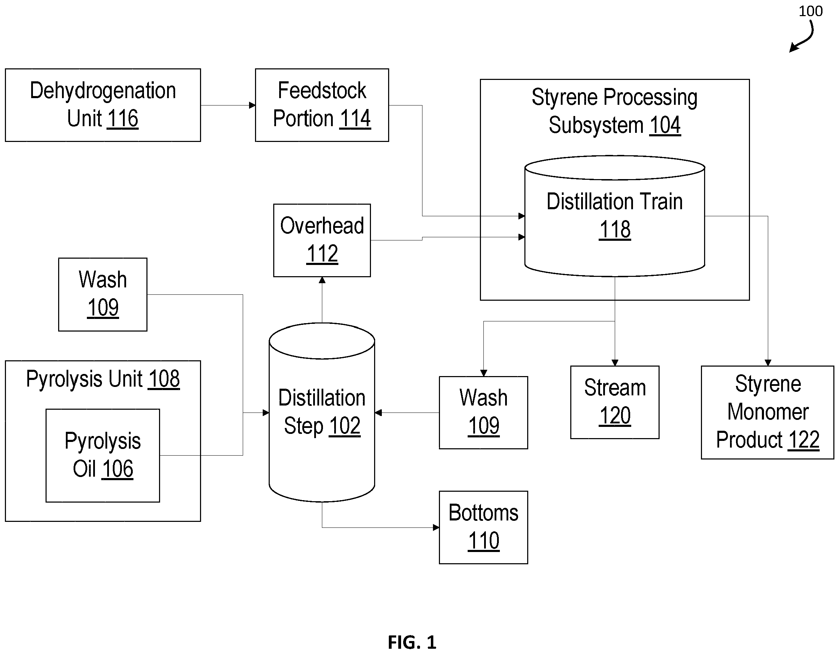

FIG. 1 is a diagram of a styrene processing system 100 that includes a distillation step 102 for separating styrene from contaminants included in pyrolysis oil according to some examples of the present disclosure. As illustrated in FIG. 1, the styrene processing system 100 can include the distillation step 102 and a styrene processing subsystem 104, though other or additional suitable columns, subsystems, and the like are possible for the styrene processing system 100. The distillation step 102 may be or include a first distillation column that can receive pyrolysis oil 106 from a pyrolysis operation performed by a pyrolysis unit 108. The pyrolysis unit 108 may be arranged to receive waste polystyrene, or other suitable waste plastics, and to perform a pyrolysis operation to generate the pyrolysis oil 106 that can include styrene and contaminants.

The distillation step 102 can be coupled, such as via one or more pipes or other conduits, pumps, and the like, to the pyrolysis unit 108 to receive the pyrolysis oil 106 as input. In some examples, the distillation step 102 can be or include a stripper column that can cause lighter phases of material to vaporize, or otherwise separate, from heavier phases of the material. For example, the distillation step 102 may receive the pyrolysis oil 106 and may strip the pyrolysis oil 106 to cause styrene, and other light or volatile materials, included in the pyrolysis oil 106 to separate from contaminants, or other heavy or non-volatile materials, included in the pyrolysis oil 106. The distillation step 102 can be used to otherwise suitably pre-process the pyrolysis oil 106 prior to providing output to the styrene processing subsystem 104.

The distillation step 102 can cause the separated styrene and contaminants to be produced, removed as residue, and the like. Removing a material, such as the contaminants or other heavy or non-volatile materials, as residue may involve removing the material from the styrene processing system 100 to be used in a separate process not involving the styrene processing system 100, to be used in a separate process within the styrene processing system 100, to be used as fuel, and the like.

In some examples, the distillation step can additionally receive wash 109, which may be or include a liquid stream, such as reflux or a slip stream, in which at least a portion of the liquid stream includes styrene. The slip stream may originate from one or more distillation columns of the styrene processing subsystem 104, may be or include a portion of styrene monomer product generated by the styrene processing system 100, may be or include a crude styrene stream from a dehydrogenation operation, may be or include a stream of styrene having light cuts removed, and the like. The wash 109 may assist in the recovery of styrene from pyrolysis oil 106 and rejection of heavy or non-volatile materials. The distillation step can receive the pyrolysis oil 106 and the wash 109 and can generate bottoms 110 and overhead 112.

The bottoms 110 may include the contaminants or other heavy or non-volatile materials separated from the pyrolysis oil 106. In some examples, the bottoms 110 may include carbon-based material heavier than carbon-based material having nine carbons (*C*₉), and the overhead 112 may include carbon-based material lighter than, or having approximately the same volatility as, carbon-based material having nine carbons (*C*₉). For example, the bottoms 110 may include *C*₁₀-based carbon material, *C*₁₁-based carbon material, *C*₁₂-based carbon material, and so on. Additionally, or alternatively, the overhead 112 may include the styrene and other light or volatile materials separated from the pyrolysis oil 106. In some examples, the overhead 112 can include *C*₈-based carbon material, *C*₇-based carbon material, *C*₆-based carbon material, and so on. In some examples, the bottoms 110, the overhead 112, or a combination thereof may include trace amounts of *C*₉-based carbon-based material. The bottoms 110 can be produced or otherwise removed as residue to be used in other processes, to be used as fuel, and the like.

The overhead 112, which includes styrene, can be provided to the styrene processing subsystem 104 as at least a portion of feedstock into the styrene processing subsystem 104. In some examples, and along with the feedstock portion 114 from a dehydrogenation unit 116, the styrene processing subsystem 104 can additionally receive the overhead 112 as feedstock. The dehydrogenation unit 116 may generate, for example by dehydrogenating ethylbenzene, styrene or crude styrene, which may include styrene and trace contaminants, as the feedstock portion 114. As illustrated in FIG. 1, the dehydrogenation unit 116 is separate from the styrene processing subsystem 104. In some examples, the dehydrogenation unit 116 may be included in or otherwise coupled with the styrene processing subsystem 104. For example, the dehydrogenation unit 116 and the styrene processing subsystem 104 may form a styrene processing system that can be augmented with the distillation step 102 to allow the styrene processing system to use feedstock from the pyrolysis unit 108. The styrene processing subsystem 104 can receive the overhead 112 from the distillation step 102 and the feedstock portion 114 from the dehydrogenation unit 116.

In some examples, the styrene processing subsystem 104 can include a distillation train 118 that can include one or more distillation steps that can each involve a distillation column. For example, the distillation train 118 can include one distillation column, two distillation columns, three distillation columns, four distillation columns, or more than four distillation columns. Each distillation column of the distillation train 118 can be arranged to receive increasingly purified input and to generate outputs, including increasingly purified styrene and contaminants separated from the increasingly purified styrene in which the contaminants can include carbon-based material heavier than, or lighter than, styrene, and the like. For example, a first distillation step of the distillation train 118 can use a first distillation column to receive the overhead 112 and the feedstock portion 114 and can generate intermediate outputs including overhead with contaminants, which can form a portion of the stream 120, and bottoms including at least partially purified styrene by distilling the combination of the overhead 112 and the feedstock portion 114. In some examples, the distillation steps of the distillation train 118 may generate a styrene stream via bottoms by separating contaminants lighter, or more volatile, than styrene via overhead, or the distillation steps may generate a styrene stream via overhead by separating contaminants heavier, or less volatile, than styrene. A subsequent, such as an intermediate or final, distillation step of the distillation train 118 can receive the intermediate outputs and generate further intermediate, or final, styrene output and another portion of the stream 120, and so on. The final output generated from the distillation train 118 may be or include styrene monomer product 122 that can be produced for use in manufacturing plastics, manufacturing rubber, and the like.

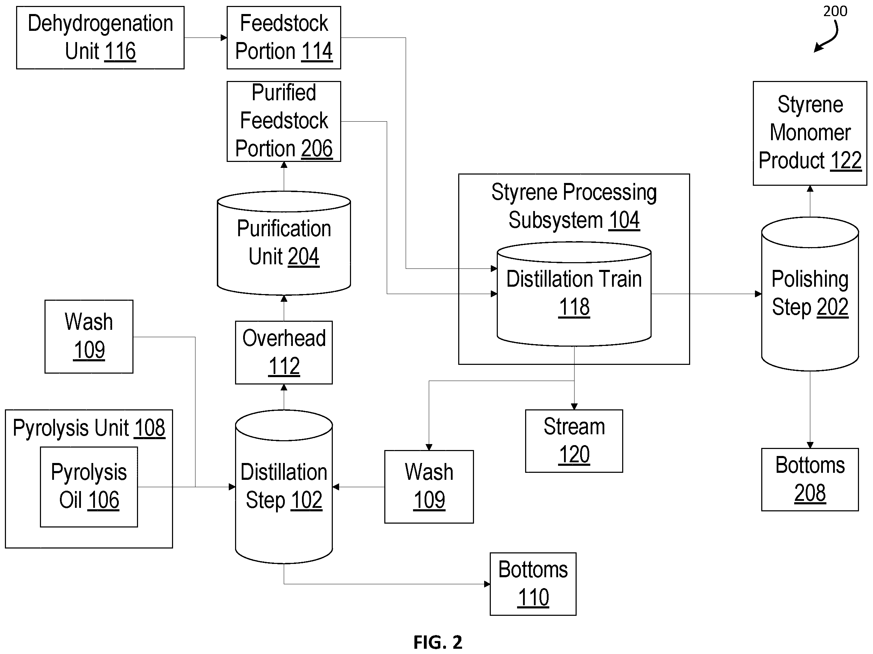

FIG. 2 is a diagram of a styrene processing system 200 that includes a polishing step 202 and a purification step 204 for generating styrene monomer product 122 according to some examples of the present disclosure. Purification step 204 may involve a distillation column, a crystallization operation, a membrane separation operation, or other purification operation to further remove contaminants from styrene. As illustrated in FIG. 2, the styrene processing system 200 can include the distillation step 102, the styrene processing subsystem 104, the polishing step 202, and the purification step 204, though other or additional suitable columns, units, subsystems, and the like are possible for the styrene processing system 200. In some examples, the polishing step 202 or the purification step 204 may be optional for including in the styrene processing system 200.

The distillation step 102 can be coupled, such as via one or more pipes or other conduits, pumps, and the like, to the pyrolysis unit 108 to perform a distillation step that can receive the pyrolysis oil 106 as input. In some examples, the distillation step 102 can be or include a stripper column that can perform the distillation step to cause lighter phases of material to vaporize, or otherwise separate, from heavier phases of the material. For example, the distillation step 102 may receive the pyrolysis oil 106 and may strip the pyrolysis oil 106 to cause styrene, and other light or volatile materials, included in the pyrolysis oil 106 to separate from contaminants, or other heavy or non-volatile materials, included in the pyrolysis oil 106. The distillation step can cause the separated styrene and contaminants to be produced, removed as residue, or the like. Removing a material, such as the contaminants or other heavy or non-volatile materials, as residue may involve removing the material from the styrene processing system 200, or otherwise transferring the material, to be used in a separate process not involving the styrene processing system 200, to be used in a separate process within the styrene processing system 200, to be used as a fuel source, and the like.

In some examples, the distillation step 102 of the distillation step can additionally receive wash 109, which may be or include a liquid stream, such as a slip stream, in which at least a portion of the liquid stream includes styrene. The slip stream may originate from one or more distillation columns of the styrene processing subsystem 104, may be or include a portion of styrene monomer product generated by the styrene processing system 100, may be or include a crude styrene stream from a dehydrogenation operation, may be or include a stream of styrene having light cuts removed, and the like. The wash 109 may assist in the recovery of styrene from pyrolysis oil 106 and rejection of heavy or non-volatile materials. The distillation step can receive the pyrolysis oil 106 and the wash 109 and can generate bottoms 110 and overhead 112.

The bottoms 110 may include the contaminants or other heavy or non-volatile materials separated from the pyrolysis oil 106. In some examples, the bottoms 110 may include carbon-based material heavier than carbon-based material having nine carbons (*C*₉), and the overhead 112 may include carbon-based material as volatile as or lighter than carbon-based material having nine carbons (*C*₉). For example, the bottoms 110 may include *C*₁₀-based carbon material, *C*₁₁-based carbon material, *C*₁₂-based carbon material, and so on. Additionally, or alternatively, the overhead 112 may include the styrene and other light or volatile materials separated from the pyrolysis oil 106. In some examples, the overhead 112 can include *C*₈-based carbon material, *C*₇-based carbon material, *C*₆-based carbon material, and so on. In some examples, the bottoms 110, the overhead 112, or a combination thereof may include trace amounts of *C*₉-based carbon-based material. The bottoms 110 can be produced or otherwise removed as residue to be used in other processes, to be used as fuel, and the like.

In some examples, the overhead 112, which includes styrene among potential contaminants, can be provided to the purification step 204. The purification step 204 can use the overhead 112 to perform a purification operation that can further remove the potential contaminants remaining in the overhead 112. For example, the purification step 204 can receive the overhead 112 and can generate a purified feedstock portion 206 that can include further-refined styrene compared with the overhead 112, with impurities such as benzene, toluene, ethylbenzene, cumene, alpha-methylstyrene, xylene, or any combination thereof reduced to significantly smaller composition fractions depending on the purification method used in portion 206 compared to the overhead 112.

The purified feedstock portion 206 can be provided to the styrene processing subsystem 104 as at least a portion of feedstock into the styrene processing subsystem 104. In some examples, the styrene processing subsystem 104 can, in addition to the feedstock portion 206, also receive the purified feedstock portion 206. As illustrated in FIG. 2, the dehydrogenation unit 116 is separate from the styrene processing subsystem 104. In some examples, the dehydrogenation unit 116 may be included in or otherwise coupled with the styrene processing subsystem 104. For example, the dehydrogenation unit 116 and the styrene processing subsystem 104 may form a styrene processing system that can be augmented with the distillation column 102 to allow the styrene processing system to use feedstock from the pyrolysis unit 108. The styrene processing subsystem 104 can receive the purified feedstock portion 206 from the purification step 204 and the feedstock portion 114 from the dehydrogenation unit 116.

In some examples, the styrene processing subsystem 104 can include a distillation train 118 that can include one or more distillation steps that can each involve a distillation column. For example, the distillation train 118 can include one distillation column, two distillation columns, three distillation columns, four distillation columns, or more than four distillation columns. Each distillation column of the distillation train 118 can be arranged to receive increasingly purified input and to generate outputs, including increasingly purified styrene and contaminants separated from the increasingly purified styrene in which the contaminants can include carbon-based material heavier than, or lighter than, styrene, and the like. For example, a first distillation step of the distillation train 118 can use a first distillation column to receive the purified feedstock portion 206 and the feedstock portion 114 and can generate intermediate outputs including overhead with contaminants, which can form a portion of the stream 120, and bottoms including at least partially purified styrene by distilling the combination of the purified feedstock portion 206 and the feedstock portion 114. In some examples, the distillation steps of the distillation train 118 may generate a styrene stream via bottoms by separating contaminants lighter, or more volatile, than styrene via overhead, or the distillation steps may generate a styrene stream via overhead by separating contaminants heavier, or less volatile, than styrene. A subsequent, such as an intermediate or final, distillation step of the distillation train 118 can receive the intermediate outputs and generate further intermediate, or final, styrene output and another portion of the stream 120, and so on. The final output generated from the distillation train 118 may be or include styrene monomer product 122 that can be produced for use in manufacturing plastics, manufacturing rubber, and the like.

In some examples, the final output generated from the distillation train 118 may be provided to the polishing step 202. The polishing step 202 may be or include a polishing column, a distillation column, a stripping column, or the like. The polishing step may further refine or distill the final output provided by the distillation train 118 to generate the styrene monomer product 122. In some examples, the polishing step 202 can generate overhead, which can be or include the styrene monomer product 122, and bottoms 208, which can include contaminants, such as non-volatile or heavy carbon-based material, separated from the final overhead. The styrene monomer product 122 may include a first concentration or purity of styrene that is higher than a second concentration or purity of styrene of the final overhead produced by the distillation train 118.

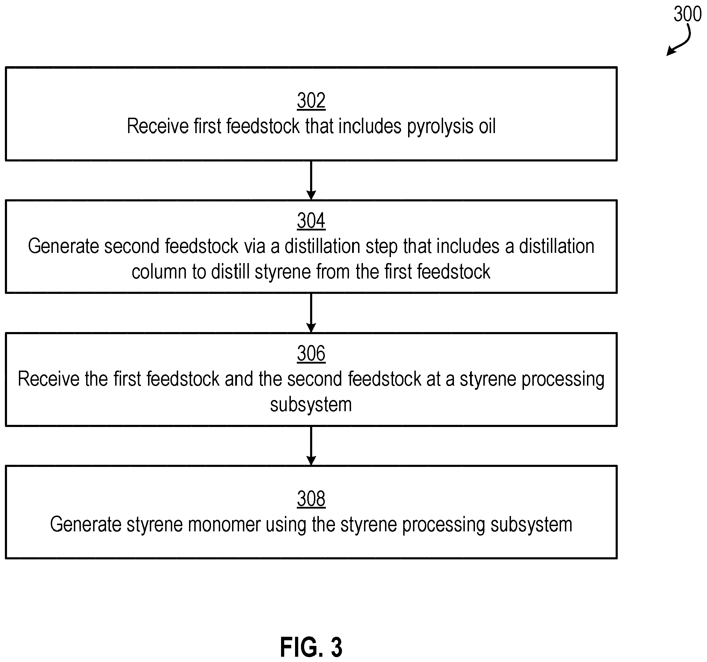

FIG. 3 is a flowchart of a process 300 for generating styrene monomer using a styrene processing system 100 that includes a distillation step for separating styrene from contaminants included in pyrolysis oil 106 according to some examples of the present disclosure. At block 302, first feedstock is received. The first feedstock may be or include the pyrolysis oil 106, and the first feedstock may be received at or otherwise by the distillation step. In some examples, the pyrolysis oil 106 may be generated by a pyrolysis unit 108 that can receive waste polystyrene, or other suitable waste plastics, and that can generate the pyrolysis oil 106. The pyrolysis unit 108 can strip styrene from the pyrolysis oil 106 or can otherwise suitably separate styrene from contaminants included in the waste polystyrene or other waste plastics. The pyrolysis oil 106 can include styrene and contaminants that are separated from the styrene. In some examples, the distillation step may additionally receive wash 109, which may be or include a liquid stream, such as reflux or a slip stream, etc., in which at least a portion of the liquid stream can include styrene. For example, the wash 109 may be or include a slip stream from one or more distillation columns of a styrene processing subsystem 104, may be or include a portion of styrene monomer product generated by a styrene processing system, may be or include a crude styrene stream from a dehydrogenation operation, may be or include a stream of styrene having light cuts removed, and the like.

At block 304, at least a portion of a second feedstock is generated. The distillation step can perform a distillation operation on the first feedstock which can include the pyrolysis oil 106, the wash 109, or a combination thereof, to generate the second feedstock. The distillation operation can include separating the first feedstock into overhead 112 and bottoms 110. The overhead 112 can be or include the second feedstock.

The bottoms 110 may include the contaminants or other heavy or non-volatile materials separated from the pyrolysis oil 106. In some examples, the bottoms 110 may include carbon-based material heavier than *C*₉-based carbon material, and the second feedstock may include carbon-based material lighter than *C*₉-based carbon material. For example, the bottoms 110 may include *C*₁₀-based carbon material, *C*₁₁-based carbon material, *C*₁₂-based carbon material, and so on. Additionally, or alternatively, the second feedstock may include the styrene and other light or volatile materials separated from the first feedstock. In some examples, the second feedstock can include *C*₈-based carbon material, *C*₇-based carbon material, *C*₆-based carbon material, and so on. In some examples, the bottoms 110, the second feedstock, or a combination thereof may include negligible or trace amounts of *C*₉-based carbon-based material. The bottoms 110 can be produced or otherwise removed as residue to be used in other processes, to be used as fuel, and the like. In some examples, the second feedstock may be provided to the styrene processing subsystem 104 for further processing or refining such as to generate a styrene monomer product 122.

At block 306, the first feedstock and the second feedstock are received at the styrene processing subsystem 104. The styrene processing subsystem 104 may include a distillation train 118 that can include one or more distillation steps that can be used to further distill or otherwise refine the first feedstock and the second feedstock into the styrene monomer product 122. The first feedstock and the second feedstock can be provided to a first distillation step of the distillation train 118. For example, the distillation step 102 may be at least fluidically coupled with the first distillation step of the distillation train 118, and the overhead 112, such as the second feedstock, of the distillation step 102 can be transferred to the first distillation step of the distillation train 118. Additionally or alternatively, the first distillation column can be at least fluidically coupled with a dehydrogenation unit 116 that can provide feedstock portion 114 that can include styrene, among trace contaminants. The feedstock portion 114 can be or include the first feedstock and can be provided to the first distillation column.

At block 308, the styrene monomer product 122 is produced. The styrene processing subsystem 104 can generate the styrene monomer product 122 using the distillation train 118. For example, each distillation column of the distillation train 118 can be arranged to receive increasingly purified input and to generate output, including increasingly purified styrene, and stream 120 that can include contaminants, carbon-based material heavier or lighter than styrene, and the like and that can be removed as residue. For example, a first distillation step of the distillation train 118 can receive the first feedstock and the second feedstock and can generate intermediate output and a portion of the stream 120 by distilling the first feedstock and the second feedstock, and so on. The final output generated from the distillation train 118 may be or include styrene monomer product 122 that can be produced for use in manufacturing plastics, manufacturing rubber, and the like.

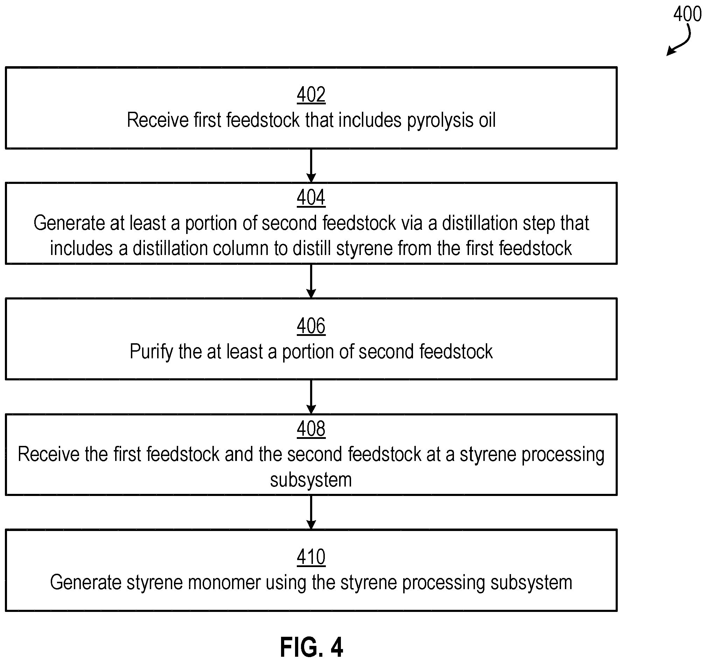

FIG. 4 is a flowchart of a process for generating styrene monomer using a styrene processing system that includes a polishing step and a purification step according to some examples of the present disclosure. At block 402, first feedstock is received. The first feedstock may be or include the pyrolysis oil 106, and the first feedstock may be received at or otherwise by the distillation step. In some examples, the pyrolysis oil 106 may be generated by a pyrolysis unit 108 that can receive waste polystyrene, or other suitable waste plastics, and that can generate the pyrolysis oil 106. The pyrolysis unit 108 can strip styrene from the pyrolysis oil 106 or can otherwise suitably separate styrene from contaminants included in the waste polystyrene or other waste plastics. The pyrolysis oil 106 can include styrene and contaminants that are separated from the styrene. In some examples, the distillation step may additionally receive wash 109, which may be or include a liquid stream in which at least a portion of the liquid stream can include styrene. For example, the wash 109 may be or include a slip stream from one or more distillation steps of a styrene processing subsystem 104, may be or include a portion of styrene monomer product generated by a styrene processing system, may be or include a crude styrene stream from a dehydrogenation operation, may be or include a stream of styrene having light cuts removed, and the like.

At block 404, at least a portion of a second feedstock is generated. The distillation step can perform a distillation operation on the first feedstock which can include the pyrolysis oil 106, the wash 109, or a combination thereof, to generate the second feedstock. The distillation operation can include separating the first feedstock into overhead 112 and bottoms 110. The overhead 112 can be or include the second feedstock.

The bottoms 110 may include the contaminants or other heavy or non-volatile materials separated from the pyrolysis oil 106. In some examples, the bottoms 110 may include carbon-based material heavier than *C*₉-based carbon material, and the second feedstock may include carbon-based material of equal volatility or lighter than *C*₉-based carbon material. For example, the bottoms 110 may include *C*₁₀-based carbon material, *C*₁₁-based carbon material, *C*₁₂-based carbon material, and so on. Additionally, or alternatively, the second feedstock may include the styrene and other light or volatile materials separated from the first feedstock. In some examples, the second feedstock can include *C*₈-based carbon material, *C*₇-based carbon material, *C*₆-based carbon material, and so on. In some examples, the bottoms 110, the second feedstock, or a combination thereof may include negligible or trace amounts of *C*₉-based carbon-based material. The bottoms 110 can be produced or otherwise removed as residue to be used in other processes, to be used as fuel, and the like. In some examples, the second feedstock may be provided to a purification step 204 for further processing, refining, or the like.

At block 406, the second feedstock provided to the purification step 204 to further remove contaminants from styrene. In some examples, the second feedstock, which includes styrene among other potential contaminants, can be provided to the purification step 204. The purification step 204 can use the second feedstock to perform a purification operation that can further remove the potential contaminants remaining in the second feedstock. For example, the purification step 204 can receive the second feedstock and can generate a feedstock portion 206, or second feedstock, that can include further-refined styrene with lower concentrations of benzene, toluene, ethylbenzene, cumene, alpha-methylstyrene, xylene, or any combination thereof compared with the second feedstock.

At block 408, the first feedstock and the second feedstock are received at the styrene processing subsystem 104. The styrene processing subsystem 104 may include a distillation train 118 that can include one or more distillation steps that can be used to further distill or otherwise refine the first feedstock and the second feedstock into the styrene monomer product 122. The first feedstock and the second feedstock can be provided to a first distillation step of the distillation train 118. For example, the purification step 204 may be at least fluidically coupled with the first distillation step of the distillation train 118, and the second feedstock of the purification step 204 can be transferred to the first distillation step of the distillation train 118. Additionally or alternatively, the first distillation step can be at least fluidically coupled with a dehydrogenation unit 116 that can provide feedstock portion 114, which may be the first feedstock, that can include crude styrene, among trace contaminants.

At block 410, the styrene monomer product 122 is produced. The styrene processing subsystem 104 can generate the styrene monomer product 122 using the distillation train 118. For example, each distillation step of the distillation train 118 can be arranged to receive increasingly purified input and to generate output, including increasingly purified styrene, and stream 120 that can include contaminants, carbon-based material heavier or lighter than styrene, and the like and that can be removed as residue. For example, a first distillation step of the distillation train 118 can receive the first feedstock and the second feedstock and can generate intermediate output and a portion of the stream 120 by distilling the first feedstock and the second feedstock, and so on. The final output generated from the distillation train 118 may be or include styrene monomer product 122 that can be produced for use in manufacturing plastics, manufacturing rubber, and the like.

The following is a set of examples of the present disclosure, though other examples are possible. As used below, any reference to a series of examples is to be understood as a reference to each of those examples disjunctively (e.g., "Examples 1-4" is to be understood as "Examples 1, 2, 3, or 4").

Example 1 is a system comprising: a styrene processing subsystem positionable to receive first feedstock and to generate styrene monomer, the styrene processing subsystem comprising an initial distillation column; and a second distillation column couplable with the initial distillation column to provide the initial distillation column with at least a portion of the first feedstock, the second distillation column positionable to receive second feedstock comprising a pyrolysis oil and to generate the portion of the first feedstock by separating styrene from the pyrolysis oil.

Example 2 is the system of example 1, wherein the second distillation column is a stripper column, wherein the second feedstock comprises a mixture of styrene and contaminants, and wherein the second feedstock is generatable via pyrolysis of polystyrene.

Example 3 is the system of any of examples 1-2, wherein the first feedstock comprises (i) third feedstock originating from a styrene dehydrogenation process and (ii) fourth feedstock originating from a pyrolysis process, and wherein the fourth feedstock is the portion.

Example 4 is the system of any of examples 1-3, wherein first outputs and second outputs are generatable by the second distillation column, wherein the first outputs are different than the second outputs, and wherein the first outputs comprise material having carbon-based material heavier than *C*₉.

Example 5 is the system of any of examples 1 or 4, wherein the second outputs comprise material having carbon-based material of the same volatility or lighter than *C*₉.

Example 6 is the system of example 1, further comprising a purification step couplable with the second distillation column and the styrene processing subsystem to separate out at least a subset of the first feedstock prior to the first feedstock being provided to the styrene processing subsystem.

Example 7 is the system of any of examples 1 or 6, wherein the purification unit is positionable to perform the purification step that comprises receiving third outputs from the second distillation column and to provide fourth outputs generatable by the purification unit to the styrene processing subsystem, and wherein the purification step comprises a crystallization step or a membrane separation step.

Example 8 is the system of any of examples 1 or 6-7, wherein fifth outputs and sixth outputs are generatable by the second distillation column, wherein the fifth outputs are different than the sixth outputs, wherein the fifth outputs comprise material having carbon-based material heavier than *C*₉, wherein the sixth outputs comprise material having carbon-based material lighter than *C*₉, and wherein the fourth outputs are generatable by the purification unit purifying the sixth outputs.

Example 9 is a method comprising: receiving, at a first distillation column of a styrene processing system, first feedstock comprising a pyrolysis oil; generating, by the first distillation column, second feedstock by distilling styrene from the first feedstock; receiving, by a second distillation column of a styrene processing subsystem of the styrene processing system, the first feedstock and the second feedstock; and generating, by a distillation train comprising the second distillation column, styrene monomer.

Example 10 is the method of example 9, wherein the first distillation column is a stripper column, wherein the first feedstock comprises a mixture of styrene and contaminants, and wherein the first feedstock is generated via pyrolysis of polystyrene.

Example 11 is the method of any of examples 9-10, wherein the third feedstock comprises (i) fourth feedstock originating from a styrene dehydrogenation process and (ii) the second feedstock originating from a pyrolysis process.

Example 12 is the method of any of examples 9-11, wherein generating the second feedstock comprises generating first outputs and second outputs, wherein the first outputs are different than the second outputs, and wherein the first outputs comprise material having carbon-based material heavier than *C*₉.

Example 13 is the method of any of examples 9 or 12, wherein the second outputs comprise material having carbon-based material of the same volatility or lighter than *C*₉.

Example 14 is the method of example 9, further comprising purification, by a purification step of the styrene processing system, the second feedstock prior to the third feedstock being provided to the second distillation column.

Example 15 is the method of any of examples 9 or 14, wherein purifying the second feedstock comprises: receiving third outputs from the first distillation column; and providing fourth outputs generated by the purification step to the second distillation column, wherein fifth outputs and sixth outputs are generated by the first distillation column, wherein the fifth outputs are different than the sixth outputs, wherein the fifth outputs comprise material having carbon-based material heavier than *C*₉, wherein the sixth outputs comprise material having carbon-based material lighter than *C*₉, and wherein the fourth outputs are generated by the purification step purifying the sixth outputs.

Example 16 is a system comprising: a styrene processing subsystem positionable to receive first feedstock and to generate styrene monomer, the styrene processing subsystem comprising an initial distillation column and a final distillation column; a second distillation column couplable with the initial distillation column to provide the initial distillation column with at least a portion of the first feedstock, the second distillation column positionable to receive second feedstock comprising a pyrolysis oil and to generate the portion of the first feedstock by separating styrene from the pyrolysis oil; and a polishing column couplable with the final distillation column of the styrene processing subsystem to generate polished styrene monomer product using the styrene monomer.

Example 17 is the system of example 16, wherein the second distillation column is a stripper column, wherein the second feedstock comprises a mixture of styrene and contaminants, and wherein the second feedstock is generatable via pyrolysis of polystyrene.

Example 18 is the system of any of examples 16-17, wherein the first feedstock comprises (i) third feedstock originating from a styrene dehydrogenation process and (ii) fourth feedstock originating from a pyrolysis process, and wherein the fourth feedstock is the portion.

Example 19 is the system of any of examples 16-18, wherein first outputs and second outputs are generatable by the second distillation column, wherein the first outputs are different than the second outputs, wherein the first outputs comprise material having carbon-based material heavier than *C*₉, and wherein the second outputs comprise material having carbon-based material lighter than *C*₉.

Example 20 is the system of example 16, further comprising a purification step couplable with the second distillation column and the styrene processing subsystem to purify at least a subset of the first feedstock prior to the first feedstock being provided to the styrene processing subsystem.

Example 21 is the system of any of examples 16-20, wherein: the polishing column is operable (a) with a bottom temperature from approximately 50 °C to approximately 100 °C, (b) with a top or head temperature from approximately 40 °C to approximately 90 °C, (c) at a bottom pressure from approximately 40 mmHg to approximately 200 mmHg, and (d) at a top or head pressure from approximately 30 mmHg to approximately 120 mmHg; and the distillation column is operable (a) with a bottom temperature from approximately 90 °C to approximately 155 °C, (b) with a top or head temperature from approximately 55 °C to approximately 100 °C, (c) at a bottom pressure from approximately 40 mmHg to approximately 300 mmHg, and (d) at a top or head pressure from approximately 40 mmHg to approximately 180 mmHg.

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure. For instance, any examples described herein can be combined with any other examples to yield further examples.

## Claims

1. A system comprising:
a styrene processing subsystem positionable to receive first feedstock and to generate styrene monomer, the styrene processing subsystem comprising an initial distillation column; and
a second distillation column couplable with the initial distillation column to provide the initial distillation column with at least a portion of the first feedstock, the second distillation column positionable to receive second feedstock comprising a pyrolysis oil and to generate the portion of the first feedstock by separating styrene from the pyrolysis oil.

2. The system of claim 1, wherein the second distillation column is a stripper column, wherein the second feedstock comprises a mixture of styrene and contaminants, and wherein the second feedstock is generatable via pyrolysis of polystyrene.

3. The system of any of claims 1-2, wherein the first feedstock comprises two different streams comprising (i) third feedstock originating from a dehydrogenation process and (ii) fourth feedstock originating from a pyrolysis process, and wherein the fourth feedstock is the portion.

4. The system of any of claims 1-3, wherein first outputs and second outputs are generatable by the second distillation column, wherein the first outputs are different than the second outputs, wherein the first outputs comprise material having carbon-based material heavier than or including *C*₉, and wherein the second outputs comprise material having carbon-based material lighter than or including *C*₉, and wherein the second distillation column is operable (a) with a bottom temperature from approximately 90 °C to approximately 155 °C, (b) with a top or head temperature from approximately 55 °C to approximately 100 °C, (c) at a bottom pressure from approximately 40 mmHg to approximately 300 mmHg, and (d) at a top or head pressure from approximately 40 mmHg to approximately 180 mmHg..

5. The system of claim 4, wherein a polishing step is performable by a polishing column couplable with the styrene processing subsystem, wherein the polishing column is operable (a) with a bottom temperature from approximately 50 °C to approximately 100 °C, (b) with a top or head temperature from approximately 40 °C to approximately 90 °C, (c) at a bottom pressure from approximately 40 mmHg to approximately 200 mmHg, and (d) at a top or head pressure from approximately 30 mmHg to approximately 120 mmHg,

6. The system of claim 1, further comprising a purification unit couplable with the second distillation column and the styrene processing subsystem to perform a purification step to purify at least a subset of the first feedstock prior to the first feedstock being provided to the styrene processing subsystem.

7. The system of any of claims 1 or 6, wherein the purification unit is positionable to perform the purification step that comprises receiving third outputs from the second distillation column and to provide fourth outputs generatable by the purification unit to the styrene processing subsystem, and wherein the purification step comprises a crystallization step or a membrane separation step or a distillation step.

8. The system of any of claims 1 or 6-7, wherein fifth outputs and sixth outputs are generatable by the second distillation column, wherein the fifth outputs are different than the sixth outputs, wherein the fifth outputs comprise material having carbon-based material heavier than or including *C*₉, wherein the sixth outputs comprise material having carbon-based material lighter than or including *C*₉, and wherein the fourth outputs are generatable by the purification unit purifying the sixth outputs.

9. A method comprising:
receiving, at a second distillation column of a styrene processing system, a first portion of a first feedstock, the first portion comprising a pyrolysis oil;
generating, by the second distillation column, second feedstock by distilling styrene from the first feedstock;
receiving, by an initial distillation column of a styrene processing subsystem of the styrene processing system, a second portion of the first feedstock and the second feedstock; and
generating, by a distillation train comprising the initial distillation column, styrene monomer.

10. The method of claim 9, wherein the second distillation column is a stripper column, wherein the first feedstock comprises a mixture of styrene and contaminants, and wherein the first feedstock is generated via pyrolysis of polystyrene.

11. The method of any of claims 9-10, further comprising a polishing step performed by a polishing unit coupled with the distillation train, wherein the polishing step comprises further distilling the styrene monomer to generate styrene monomer product.

12. The method of any of claims 9-11, wherein generating the second feedstock comprises generating first outputs and second outputs, wherein the first outputs are different than the second outputs, and wherein the first outputs comprise material having carbon-based material heavier than or including *C*₉.

13. The method of claim 12, wherein the second outputs comprise material having carbon-based material lighter than or including *C*₉.

14. The method of claim 9, further comprising purifying, by a purification step that comprises a separate distillation column, a crystallization unit, or a membrane separation unit, the second feedstock prior to the second feedstock being provided to the initial distillation column.

15. The method of any of claims 9 or 14, wherein purifying the second feedstock comprises:
receiving third outputs from the second distillation column; and
providing fourth outputs generated by the purification step to the initial distillation column, wherein fifth outputs and sixth outputs are generated by the second distillation column, wherein the fifth outputs are different than the sixth outputs, wherein the fifth outputs comprise material having carbon-based material heavier than *C*₉, wherein the sixth outputs comprise material having carbon-based material lighter than *C*₉, and wherein the fourth outputs are generated by the purification step purifying the sixth outputs.
